(19)

(11) **EP 3 610 320 B1**

(12) **EUROPÄISCHE PATENTSCHRIFT**


(45) Veröffentlichungstag und Bekanntmachung des Hinweises auf die Patenterteilung: **19.08.2020 Patentblatt 2020/34**

(21) Anmeldenummer: **18716128.6**

(22) Anmeldetag: **20.03.2018**

(51) Int Cl.:
***G02C 5/22*** *(2006.01)* ***G02C 5/00*** *(2006.01)*
***G02C 5/18*** *(2006.01)* ***A61F 9/02*** *(2006.01)*

(86) Internationale Anmeldenummer: **PCT/EP2018/056927**

(87) Internationale Veröffentlichungsnummer: **WO 2018/188906 (18.10.2018 Gazette 2018/42)**


(54) **BRILLENBÜGEL**

EYEGLASS TEMPLE

BRANCHE DE LUNETTES


(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.04.2017 DE 102017206431**

(43) Veröffentlichungstag der Anmeldung: **19.02.2020 Patentblatt 2020/08**



(73) Patentinhaber: **Uvex Arbeitsschutz GmbH 90766 Fürth (DE)**

(72) Erfinder:
- **ANGEBRANDT, Helena 91207 Lauf (DE)**
- **KÜHNLEIN, Florian 90763 Fürth (DE)**
- **WACKER, Marco 91452 Wilhermsdorf (DE)**
- **SCHUSS, Frederic 90419 Nürnberg (DE)**

(74) Vertreter: **Rau, Schneck & Hübner Patentanwälte Rechtsanwälte PartGmbB Königstraße 2 90402 Nürnberg (DE)**



(56) Entgegenhaltungen:
**EP-A2- 0 457 026** **US-A1- 2009 059 160**
**US-A1- 2016 033 790** **US-B1- 7 165 838**



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



EP 3 610 320 B1



Printed by Jouve, 75001 PARIS (FR)

## Beschreibung

Die vorliegende Patentanmeldung nimmt die Priorität der deutschen Patentanmeldung DE 10 2017 206 431.5 in Anspruch.

Die Erfindung betrifft einen Brillenbügel für eine Brille, insbesondere für eine Arbeitsschutzbrille, gemäß dem Oberbegriff des Anspruchs 1. Sie richtet sich ferner auf eine Brille mit zwei derartigen Brillenbügeln.

Aus dem Stand der Technik sind durch offenkundige Vorbenutzung bereits Brillenbügel bekannt, die aufwändige Federscharniere mit Federn umfassen. Derartige Brillenbügel erfordern häufig eine komplizierte und aufwändige Montage.

Aus der US 7,165,838 B1, die den nächsten Stand der Technik bildet, ist eine Brille bekannt, die eine Scheibenanordnung und an diese mittels Gelenkanordnungen angelenkte Brillenbügel umfasst. Elastische Bandelemente sind innen an den Brillenbügeln angeordnet und erstrecken sich über die Gelenkanordnungen. Jedes Bandelement umfasst Kopfteile, die in entsprechende Aussparungen in dem zugehörigen Brillenbügel und der Scheibenanordnung eingreifen. Die Bandelemente drücken die Brillenbügel an den Kopf eines Brillenträgers. Diese Brille bietet häufig keinen hohen Tragekomfort. Ferner ist diese aufwändig in der Herstellung.

Die US 2016/033790 A1 offenbart eine Brille mit einem Brillenrahmen und Brillenbügeln, die jeweils mittels eines O-Rings an dem Brillenrahmen gehalten sind. Jeder O-Ring läuft um einen an dem Brillenrahmen festgelegten Stift und einen Vorsprung, der Bestandteil des jeweiligen Brillenbügels ist. Die O-Ringe erlauben eine nachgiebige Anordnung der Brillenbügel an dem Brillenrahmen.

Aus der EP 0 457 026 A2 ist eine Brille bekannt, deren Brillenbügel an einer Scheibenanordnung angelenkt sind. Jeder Brillenbügel trägt endseitig ein elastisches Gelenkelement, das an der Scheibenanordnung anliegt, wenn der Brillenbügel in eine Öffnungsstellung verschwenkt wird. Eine Verformung des elastischen Gelenkelements zwängt den Brillenbügel in seine teilweise geschlossene Stellung zurück.

Aus der US 2009/0059160 A1 ist eine Brille bekannt, die einen Brillenrahmen umfasst. An dem Brillenrahmen sind randseitig zwei Kopplungsteile angelenkt, die flexibel und elastisch sind. In jedes Kopplungsteil ist ein Brillenbügel eingesteckt.

Die US 2,761,353 offenbart Spannvorrichtungen für Brillenrahmen. Die Spannvorrichtungen sind benachbart zu Gelenkabschnitten des Brillenrahmens angeordnet. Sie sind seitlich zu Rahmenabschnitten des Brillenrahmens angeordnet und erstrecken sich innenseitig bezüglich eines Brillenbügels an einem jeweiligen Scharnierstift vorbei. Jede Spannvorrichtung umfasst einen Körper aus nachgiebigem Material und bildet einen nachgiebigen Anschlag.

Die US 2,684,014 offenbart einen Brillenrahmen mit Mitteln zum Begrenzen einer Auswärtsbewegung eines angelenkten Brillenbügels. In einem Brillenrahmen und einem Brillenbügel sind Aussparungen ausgebildet, in welchen Elemente aus einem nachgiebigen Material angeordnet sind. Bei einer bevorzugten Ausführungsform ist an jedem Brillenbügel ein Hülsenkörper aus nachgiebigem Material angeordnet, dessen Spitze zwischen einem Ende des Brillenbügels und dem Brillenrahmen angeordnet ist.

Die DE 10 2005 014 512 A1 offenbart eine Brillenfassung mit einer Backe und einem angelenkten Bügel. Zwischen der Backe und dem Bügel ist eine elastische Lippe angeordnet. Die elastische Lippe wird durch Öffnen des Bügels gegen die Backe gepresst und verformt.

Der Erfindung liegt daher die Aufgabe zugrunde, einen Brillenbügel bereitzustellen, der zum einen kostengünstig herstellbar und einfach montierbar und zum anderen besonders komfortabel für einen Träger des Brillenbügels bzw. der Brille ist. Eine entsprechende Brille soll außerdem geliefert werden.

Diese Aufgabe wird erfindungsgemäß durch die in den Ansprüchen 1 und 12 angegebenen Merkmale gelöst. Der Kern der Erfindung liegt darin, dass der Brillenbügel direkt an seinem Brillenbügel-Gelenkkörper eine Gelenckörper-Weichkomponente aufweist, die bereits dort ein federndes Verschwenken, insbesondere federndes Aufbiegen aus einer nicht-aufgebogenen bzw. ursprünglichen Brillenbügel-(Trage)stellung und/oder Rückfedern, des Brillenbügels ermöglicht. Dies erlaubt eine besonders gute und einfache Anpassung des Brillenbügels bzw. der Brille an unterschiedlich breite bzw. schmale Kopfformen von Trägern des Brillenbügels bzw. der Brille. Der Brillenbügel-Gelenkkörper selbst ist so entsprechend verformbar und bildet insbesondere einen Feder-Gelenkkörper. Die federnde Ausbildung des Brillenbügel-Gelenkkörpers bzw. der Gelenkkörper-Weichkomponente erlaubt insbesondere eine Auslenkung des Brillenbügels aus einer nicht-aufgebogenen bzw. ursprünglichen Brillenbügel-Tragestellung nach außen und veranlasst bzw. unterstützt insbesondere eine automatische/selbständige Rückführung des Brillenbügels bzw. Rückfederung in die nicht-aufgebogene bzw. ursprüngliche Brillenbügel-Tragestellung des Brillenbügels.

Insbesondere erfolgt bei Trägern mit schmalen Köpfen kein Aufbiegen des Brillenbügels unmittelbar bei dem Brillenbügel-Gelenkkörper bzw. kaum ein derartiges Aufbiegen, so dass unangenehme Druckstellen durch den Brillenbügel an dem Kopf des Trägers vermeidbar sind, aber dennoch ein sicherer Halt gegeben ist.

Wenn dagegen die Brille von einem Träger mit einem vergleichsweise breiten Kopf getragen wird, kommt es insbesondere zu einer federnden Aufbiegung bei dem Brillenbügel-Gelenkkörper. Dabei erfährt insbesondere die Weichkomponentenanordnung dort eine elastische Dehnung. Sie wirkt dieser Aufbiegung entgegen und sorgt hier für einen rückfedernden Effekt des Brillenbügels. Der Brillenbügel hat demnach an/bei dem Brillenbügel-Gelenkkörper einen integrierten Federmechanismus.

Die zusammenhängende Weichkomponentenanordnung erlaubt zum einen ein besonders vorteilhaftes Aufbiegen bzw. Rückfedern des Brillenbügels und sorgt zum anderen für einen äußerst haltbaren Brillenbügel.

Die Gelenkkörper-Weichkomponente ist bevorzugt zumindest teilweise in dem Brillenbügel-Gelenkkörper angeordnet.

Die Anordnung der Gelenkkörper-Weichkomponente in mindestens einer Brillenbügel-Gelenkkörper-Nut erlaubt eine sichere Aufnahme der Gelenkkörper-Weichkomponente in dem Brillenbügel-Gelenkkörper. Die mindestens eine Brillenbügel-Gelenkkörper-Nut hat günstigerweise einen rechteckigen Querschnitt. Sie hat vorzugsweise eine Tiefe, die zwischen 0,5 mm und 4 mm, bevorzugter zwischen 1 mm und 2,5 mm, liegt. Es ist zweckmäßig, wenn sie eine Höhe aufweist, die zwischen 0,5 mm und 4 mm, bevorzugter zwischen 1 mm und 3,5 mm, liegt.

Die Zwischen-Weichkomponente erstreckt sich günstigerweise über 10 % bis 30 % der Länge des Brillenbügel-Gelenkkörpers in der Längsrichtung des Brillenbügels. Sie ist vorzugsweise randseitig in Bezug auf den Brillenbügel-Gelenkkörper angeordnet. Es ist von Vorteil, wenn der Brillenbügel-Gelenkkörper und/oder das Brillenbügel-Hauptteil mindestens eine entsprechende Zwischen-Weichkomponenten-Aussparung für die Zwischen-Weichkomponente haben. Die Zwischen-Weichkomponente erlaubt insbesondere eine Auslenkung des Brillenbügels aus einer nicht-aufgebogenen bzw. ursprünglichen Brillenbügel-Tragestellung nach außen und veranlasst bzw. unterstützt insbesondere eine automatische/selbständige Rückführung des Brillenbügels bzw. Rückfederung in die nicht-aufgebogene bzw. ursprüngliche Brillenbügel-Tragestellung des Brillenbügels.

Die Außen-Weichkomponente ist günstigerweise in einer entsprechenden Ausnehmung in dem Brillenbügel-Hauptteil angeordnet. Es ist von Vorteil, wenn sich die Außen-Weichkomponente bis zu dem freien Ende des Brillenbügels erstreckt. Alternativ endet sie beabstandet zu dem freien Ende. Die Außen-Weichkomponente ist vorzugsweise streifenartig bzw. stegartig. Ein derartiger Brillenbügel ist insbesondere federnd ausgebildet.

Die Ausgestaltung, wonach die Zwischen-Weichkomponente und die Außen-Weichkomponente benachbart zu dem Brillenbügel-Gelenkkörper zum Ermöglichen eines, insbesondere federnden, Aufbiegens des Brillenbügels zusammenhängend sind, führt zu einem sicheren Halt der Zwischen- und Außen-Weichkomponente an dem Brillenbügel-Hauptteil.

Es ist von Vorteil, wenn der Brillenbügel beim Tragen zumindest bereichsweise seitlich an dem Kopf des Trägers anliegt. Günstigerweise liegt an der Innenseite des Brillenbügels hauptsächlich das zweite Material vor, was komfortabel ist und zu einem rutschsicheren Halt des Brillenbügels an dem Träger führt. Es ist zweckmäßig, wenn dort das zweite Material in einer entsprechenden Aufnahme bzw. Ausnehmung des Brillenbügels angeordnet ist.

Das erste Material ist günstigerweise ein vergleichsweise formstabiles Kunststoffmaterial, während das zweite Material vorzugsweise ein nachgiebiges, weiches bzw. elastisch verformbares Kunststoffmaterial ist.

Die Gelenkmittelaufnahme erlaubt insbesondere die Aufnahme eines Gelenkmittels, wie eines Stifts, Zapfens, Niets oder dergleichen, was dem Brillenbügel eine Schwenkachse vorgibt. Sie ist vorzugsweise zylindrisch ausgeführt.

Es ist von Vorteil, wenn der Brillenbügel einteilig bzw. einstückig ist. Die Brillenbügel einer Brille sind bevorzugt konstruktiv identisch ausgebildet. Das Brillenbügel-Hauptteil erstreckt sich beispielsweise gerade und/oder gekrümmt.

Die Brillen-Scheibenanordnung hat beispielsweise zwei Brillenscheiben. Alternativ hat sie zum Beispiel genau eine einstückige, durchgehende Brillenscheibe.

Weitere vorteilhafte Ausgestaltungen der Erfindung sind in den Unteransprüchen angegeben.

Die Ausbildung gemäß dem Unteranspruch 2 erlaubt eine sichere Anlenkung des Brillenbügels an der Brillen-Scheibenanordnung. Ferner ist so stets eine exakte Schwenkbewegung bzw. Führung des Brillenbügels gegeben. Die Kopffläche und Fußfläche laufen bevorzugt parallel übereinander. Sie sind vorzugsweise von der jeweiligen Gelenkmittelaufnahme durchsetzt. Die Gelenkkörper-Weichkomponente ist so, günstigerweise im Wesentlichen mittig, zwischen der Kopffläche und der Fußfläche angeordnet.

Die Ausgestaltung gemäß dem Unteranspruch 3 führt zu einem besonders guten Aufbiege- bzw. Rückfederverhalten des Brillenbügels. Die Gelenckörper-Weichkomponente erstreckt sich vorzugsweise von dem gelenkseitigen Ende des Brillenbügels in Richtung auf das gegenüberliegende freie Ende des Brillenbügels.

Die Übergangs-Weichkomponente gemäß dem Unteranspruch 4 ist günstigerweise keilförmig bzw. rampenförmig. Es ist von Vorteil, wenn sich die Übergangs-Weichkomponente von dem Brillenbügel-Gelenkkörper aus in Richtung auf das freie Ende des Brillenbügels verjüngt. Die Übergangs-Weichkomponente schließt sich vorzugsweise benachbart zu einer freien Innenseite des Brillenbügel-Gelenkkörpers an diesen an. Die Übergangs-Weichkomponente hat in der Längsrichtung des Brillenbügels bevorzugt eine Länge, die in etwa der Länge des Brillenbügel-Gelenkkörpers in der Längsrichtung des Brillenbügels entspricht. Die Übergangs-Weichkomponente erlaubt insbesondere eine Auslenkung des Brillenbügels aus einer nicht-aufgebogenen bzw. ursprünglichen Brillenbügel-Tragestellung nach außen und veranlasst bzw. unterstützt insbesondere ein automatische/selbständige Rückführung des Brillenbügels bzw. Rückfederung in die nicht-aufgebogene bzw. ursprüngliche Brillenbügel-Tragestellung des Brillenbügels.

Die Zwischen-Weichkomponente gemäß dem Unteranspruch 6 ist günstigerweise benachbart zu dem Brillenbügel-Hauptteil, an diesem und/oder in diesem angeordnet.

Die Ausgestaltung gemäß dem Unteranspruch 8 führt zu einem sicheren Halt der Zwischen- und Außen-Weichkomponente an dem Brillenbügel-Hauptteil. Die mindestens eine Verbindungsöffnung durchsetzt das Brillenbügel-Hauptteil vollständig. Sie ist vorzugsweise rechteckig.

Die Innen-Weichkomponente gemäß dem Unteranspruch 10 ist vorzugsweise streifenförmig bzw. stegartig ausgeführt. Sie ist günstigerweise gegenüberliegend zu der Außen-Weichkomponente an dem Brillenbügel-Hauptteil angeordnet. Es ist von Vorteil, wenn sich die Innen-Weichkomponente bis zu dem freien Ende des Brillenbügels erstreckt. Alternativ endet diese beanstandet zu dem freien Ende des Brillenbügels. Ein derartiger Brillenbügel ist insbesondere federnd ausgebildet.

In dem Unteranspruch 11 sind bevorzugte Härten des Brillenbügels angegeben.

Es ist von Vorteil, wenn das erste Material ein erstes Elastizitätsmodul E1 von 500 MPa bis 3.000 MPa und das zweite Material ein zweites Elastizitätsmodul E2 von 5 MPa bis 200 MPa aufweist. Vorzugsweise gilt: $2{,}5 \leq E1/E2 \leq 600$.

Es ist von Vorteil, wenn jedes Brillenbügel-Gelenk bzw. -Scharnier der Brille einen Bügelschlag zum Bewegen des jeweiligen Brillenbügels in seine benachbarte Endstellung aufweist.

Nachfolgend wird unter Bezugnahme auf die beigefügte Zeichnung eine bevorzugte Ausführungsform der Erfindung beispielhaft beschrieben. Dabei zeigen:

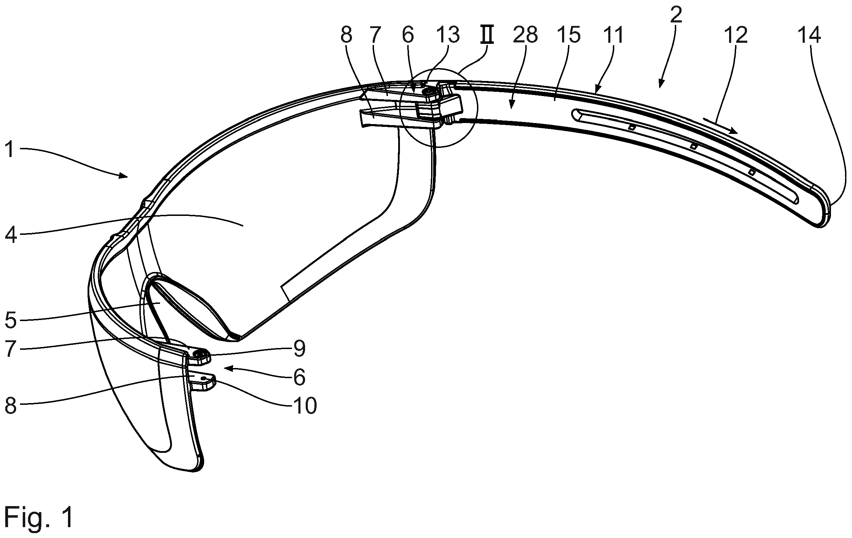

Fig. 1 eine perspektivische Ansicht einer Brille mit einem vollständig ausgeklappten erfindungsgemäßen Brillenbügel,

Fig. 2 die in Fig. 1 gekennzeichnete Einzelheit II, die ein Gelenk der Brille vergrößert veranschaulicht,

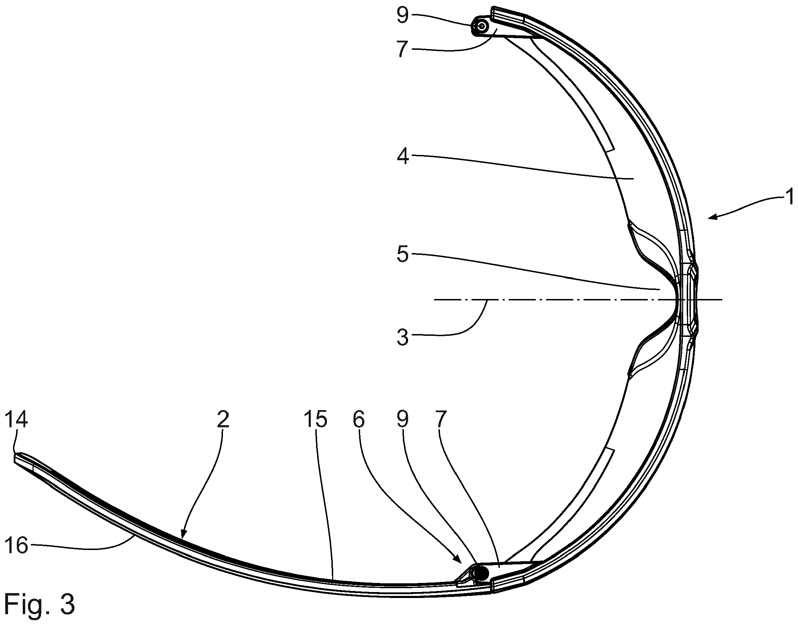

Fig. 3 eine Draufsicht auf die in Fig. 1 gezeigte Brille,

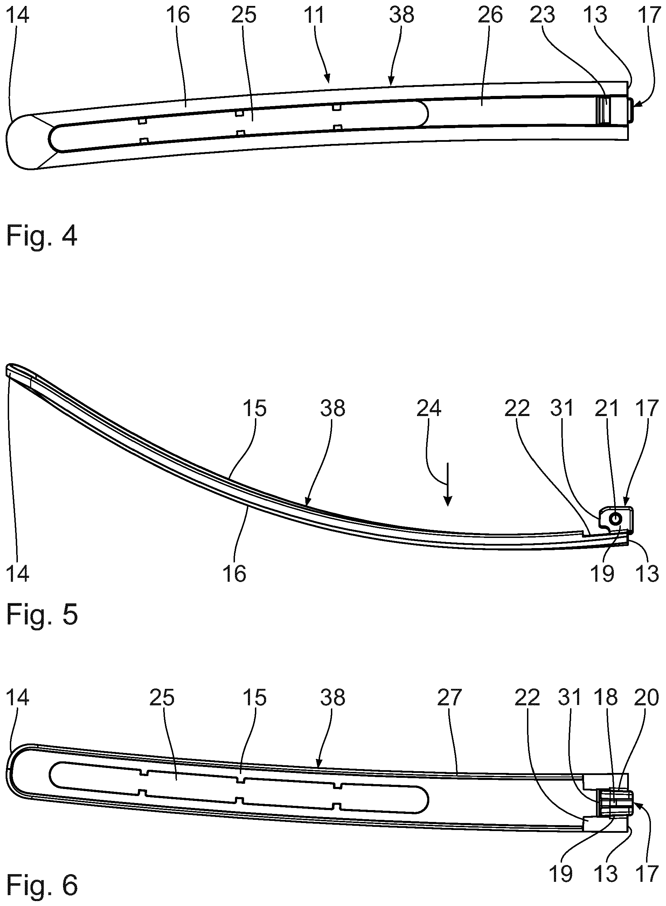

Fig. 4 eine Seitenansicht eines Brillenbügel-Grundkörpers des in den Fig. 1 und 3 gezeigten Brillenbügels von außen,

Fig. 5 eine Draufsicht auf den in Fig. 4 veranschaulichten Brillenbügel-Grundkörper,

Fig. 6 eine Seitenansicht des in Fig. 4, 5 dargestellten Brillenbügel-Grundkörpers von innen,

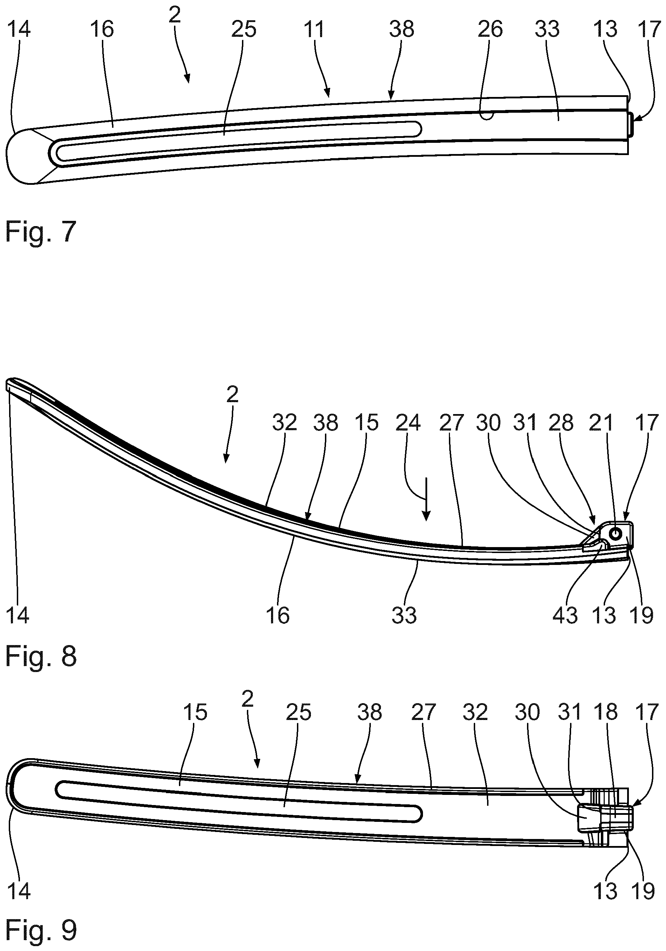

Fig. 7 eine Seitenansicht des in den Fig. 1 und 3 gezeigten fertigen Brillenbügels von außen, der den Brillenbügel-Grundkörper gemäß Fig. 4 bis 6 umfasst,

Fig. 8 eine Draufsicht auf den in Fig. 7 gezeigten fertigen Brillenbügel,

Fig. 9 eine Seitenansicht des in den Fig. 7, 8 gezeigten fertigen Brillenbügels von innen,

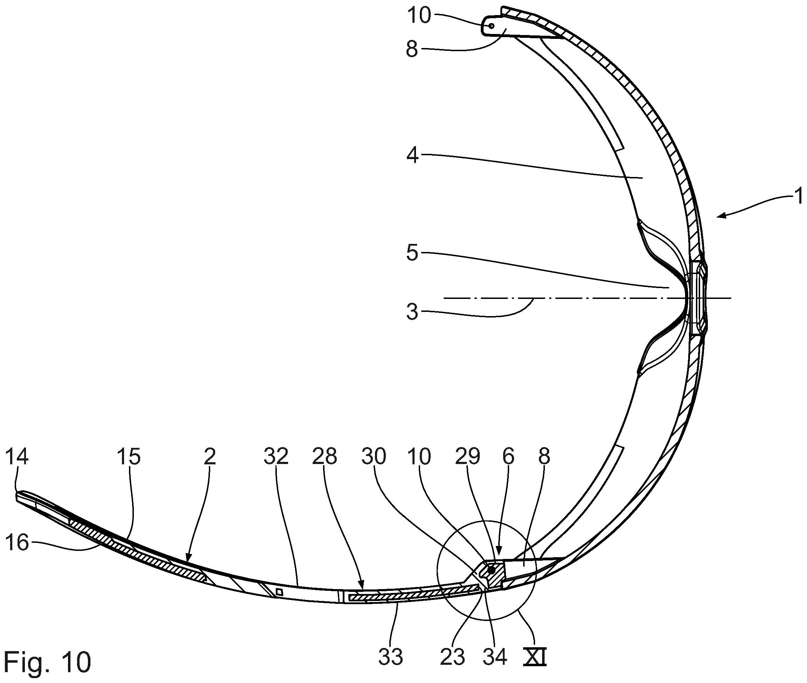

Fig. 10 eine Schnittansicht der in Fig. 1 und 3 dargestellten Brille,

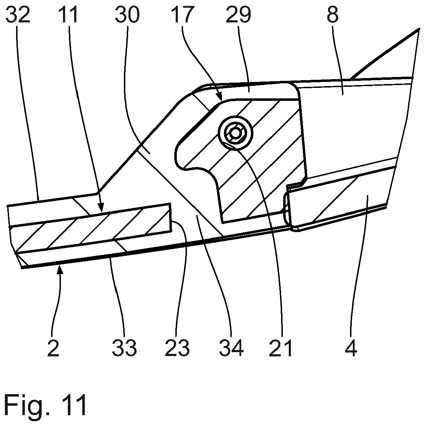

Fig. 11 die in Fig. 10 gekennzeichnete Einzelheit XI, die wieder vergrößert das Gelenk der Brille veranschaulicht,

Fig. 12 eine weitere Schnittansicht der in den Fig. 1, 3 gezeigten Brille, wobei sich der Brillenbügel in einer vollständig eingeklappten Stellung befindet,

Fig. 13 die in Fig. 12 gekennzeichnete Einzelheit XIII des Gelenks der Brille in vergrößertem Maßstab,

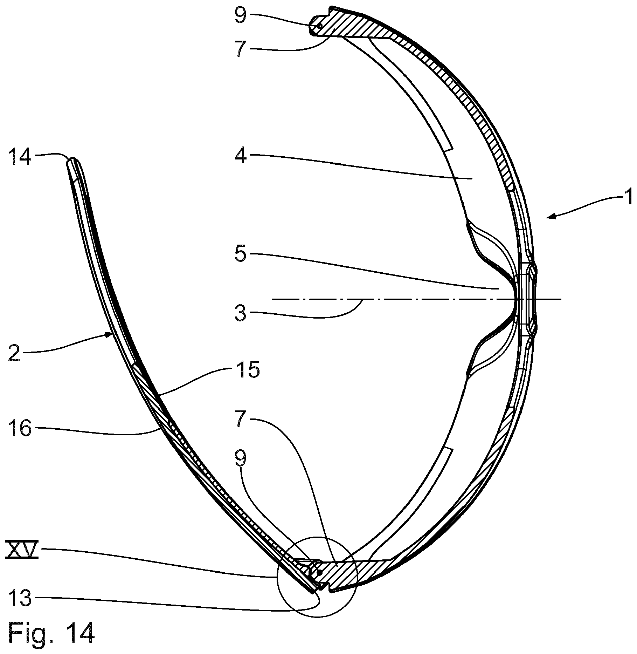

Fig. 14 eine Fig. 12 entsprechende Schnittansicht, wobei sich der Brillenbügel nun in einer teilweise eingeklappten Stellung befindet,

Fig. 15 die in Fig. 14 gekennzeichnete Einzelheit XV des Gelenks der Brille in vergrößertem Maßstab, die einen Bügelschlag veranschaulicht,

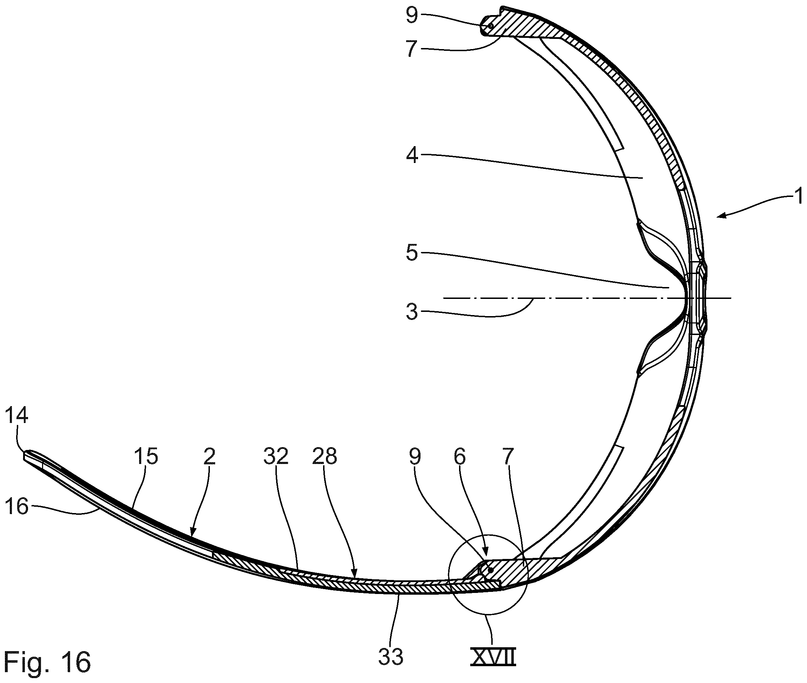

Fig. 16 eine Fig. 12 entsprechende Schnittansicht der Brille, wobei sich der Brillenbügel nun in seiner vollständig ausgeklappten Stellung befindet, und

Fig. 17 die in Fig. 16 gekennzeichnete Einzelheit XVII des Gelenks der Brille in vergrößertem Maßstab.

Eine erfindungsgemäße Brille umfasst eine Brillen-Scheibenanordnung 1 und zwei an der Brillen-Scheibenanordnung 1 seitlich angelenkte Brillenbügel 2. In den Figuren ist der Einfachheit halber nur ein Brillenbügel 2 dargestellt. Die Brille ist bezüglich einer Symmetrieebene 3 symmetrisch, die mittig durch die Brillen-Scheibenanordnung 1 läuft.

Die Brillen-Scheibenanordnung 1 hat eine einzige, durchgehende Brillenscheibe 4. In der Brillen-Scheibenanordnung 1 ist eine zentrale Nasenaufnahme 5 zur Aufnahme einer Nase eines Trägers (nicht dargestellt) ausgebildet.

Die Brillen-Scheibenanordnung 1 umfasst ferner zwei beabstandet zueinander angeordnete, seitliche Brillenbügel-Lageranordnungen 6. Die Brillenbügel-Lageranordnungen 6 sind innenseitig an der Brillen-Scheibenanordnung 1 angeordnet. Sie sind konstruktiv identisch.

Jede Brillenbügel-Lageranordnung 6 hat einen oberen ersten Scharnieransatz 7 und einen beabstandet zu diesem angeordneten, unteren zweiten Scharnieransatz 8. Der erste Scharnieransatz 7 und der zweite Scharnieransatz 8 verlaufen beabstandet übereinander unter Bildung einer Brillenbügel-Gelenkkörper-Aufnahme. Die Scharnieransätze 7, 8 der Brillenbügel-Lageranordnungen 6 springen in Richtung auf die Symmetrieebene 3 vor bzw. aufeinander zu. Sie erstrecken sich parallel zueinander.

In jedem ersten Scharnieransatz 7 ist eine zylindrische erste Lageröffnung 9 ausgebildet, die durchgängig ist. In jedem zweiten Scharnieransatz 8 ist eine zylindrische zweite Lageröffnung 10 ausgebildet, die mit der ersten Lageröffnung 9 der jeweiligen Brillenbügel-Lageranordnung 6 fluchtet und beispielsweise durchgängig ist.

Jeder Brillenbügel 2 hat einen vergleichsweise formstabilen, aber dennoch verformbaren Brillenbügel-Grundkörper 11, der sich längs einer jeweiligen Brillenbügel-Hauptrichtung 12 erstreckt und einstückig aus einem ersten Kunststoffmaterial gebildet ist.

Ferner weist jeder Brillenbügel 2 ein gelenkseitiges Ende 13 und ein freies Ende 14 auf. Das gelenkseitige Ende 13 und das freie Ende 14 des jeweiligen Brillenbügels 2 sind in der Brillenbügel-Hauptrichtung 12 zueinander beabstandet. In montiertem Zustand der Brille ist das gelenkseitige Ende 13 benachbart zu der jeweiligen Brillenbügel-Lageranordnung 6 angeordnet.

Jeder Brillenbügel 2 hat ferner eine Innenseite 15 und eine der Innenseite 15 gegenüberliegende Außenseite 16. In vollständig ausgeklapptem Zustand des montierten Brillenbügels 2 ist die Innenseite 15 dieses Brillenbügels 2 der Symmetrieebene 3 zugewandt, während die Außenseite 16 der Symmetrieebene 3 abgewandt ist. Die Innenseite 15 ist dann auch dem Kopf des Trägers zugewandt, wenn die Brille bestimmungsgemäß getragen wird.

Jeder Brillenbügel-Grundkörper 11 hat ein längliches Brillenbügel-Hauptteil 38 und einen endseitigen Brillenbügel-Gelenkkörper 17, der innenseitig an dem Brillenbügel-Hauptteil 38 benachbart zu dem zugehörigen gelenkseitigem Ende 13 angeordnet ist.

Jeder Brillenbügel-Gelenkkörper 17 ist im Wesentlichen blockartig ausgeführt und hat eine Brillenbügel-Gelenkkörper-Nut 18, die an der Innenseite 15 des Brillenbügels 2 offen ist und beabstandet zu einer oberen Kopffläche 19 und zu einer unteren Fußfläche 20 des jeweiligen Brillenbügel-Gelenkkörpers 17 gerade verläuft. Die Brillenbügel-Gelenkkörper-Nut 18 erstreckt sich im Wesentlichen in der Brillenbügel-Hauptrichtung 12.

In jedem Brillenbügel-Gelenkkörper 17 ist eine Gelenkmittelaufnahme 21 ausgebildet, die zwischen der Kopffläche 19 und der Fußfläche 20 verläuft und diese durchsetzt. Jede Gelenkmittelaufnahme 21 ist zylindrisch ausgeführt und verläuft beabstandet zu der jeweiligen Brillenbügel-Gelenkkörper-Nut 18. Jede Gelenkmittelaufnahme 21 ist ferner beabstandet zu dem gelenkseitigen Ende 13 angeordnet. Sie ist jeweils in Umfangsrichtung geschlossen und verläuft senkrecht zu der Brillenbügel-Gelenkkörper-Nut 18.

In jedem Brillenbügel-Hauptteil 38 ist benachbart zu dem jeweiligen Brillenbügel-Gelenkkörper 17 eine Zwischen-Weichkomponenten-Aussparung 22 ausgebildet, die sich auch in dem zugehörigen Brillenbügel-Gelenkkörper 17, abgewandt zu dem gelenkseitigen Ende 13 erstreckt.

In jedem Brillenbügel-Hauptteil 38 ist benachbart zu dem jeweiligen Brillenbügel-Gelenkkörper 17 eine Verbindungsöffnung 23 angeordnet, die das Brillenbügel-Hauptteil 38 vollständig in einer Brillenbügel-Querrichtung 24 senkrecht zu der Brillenbügel-Hauptrichtung 12 durchsetzt und umfangsseitig geschlossen ist.

Jedes Brillenbügel-Hauptteil 38 hat vorzugsweise eine schlitzartige Durchgangsaussparung 25, die sich längs der Brillenbügel-Hauptrichtung 12 erstreckt und umfangsseitig geschlossen ist. Jede Durchgangsaussparung 25 verläuft beabstandet zu dem Brillenbügel-Gelenkkörper 17 in Richtung auf das freie Ende 14 und ist günstigerweise beabstandet zu dem freien Ende 14 angeordnet.

Jedes Brillenbügel-Hauptteil 38 hat eine Verbindungsnut 26, die sich von dem jeweiligen Brillenbügel-Gelenkkörper 17 bis zu der zugehörigen Durchgangsaussparung 25 erstreckt und zu der Außenseite 16 des jeweiligen Brillenbügels 2 hin offen ist.

Jedes Brillenbügel-Hauptteil 38 hat ferner innenseitig einen randseitigen Umrandungssteg 27, der in der Brillenbügel-Querrichtung 24 vorspringt.

Jeder Brillenbügel 2 hat eine zusammenhängende Weichkomponentenanordnung 28, die an dem Brillenbügel-Grundkörper 11 fest angeordnet bzw. an diesen angespritzt ist. Jede Weichkomponentenanordnung 28 ist aus einem zweiten Kunststoffmaterial gebildet, das weich, elastisch bzw. federnd ist. Der Brillenbügel-Grundkörper 11 trägt somit die jeweilige Weichkomponentenanordnung 28.

Jede Weichkomponentenanordnung 28 weist eine Gelenkkörper-Weichkomponente 29 auf, die die jeweilige Brillenbügel-Gelenkkörper-Nut 18 vollständig ausfüllt, sodass der Brillenbügel-Gelenkkörper 17 federnd verformbar ist.

Ferner hat jede Weichkomponentenanordnung 28 eine Übergangs-Weichkomponente 30, die sich an einen dem gelenkseitigen Ende 13 des Brillenbügels 2 abgewandten Endbereich 31 des Brillenbügel-Gelenkkörpers 17 anschließt und von dort sich verjüngend in Richtung auf das Brillenbügel-Hauptteil 38 verläuft. Jede Übergangs-Weichkomponente 30 schließt sich dabei an die Kopffläche 19 und Fußfläche 20 des Brillenbügel-Gelenkkörpers 17 an und hat somit eine größere Höhe als die benachbarte Gelenkkörper-Weichkomponente 29. Die Gelenkkörper-Weichkomponente 29 und die Übergangs-Weichkomponente 30 des jeweiligen Brillenbügels 2 sind direkt miteinander verbunden. Sie haben in der Brillenbügel-Hauptrichtung 12 eine im Wesentlichen identische Länge. Jeder Brillenbügel 2 ist so bei der Übergangs-Weichkomponente 30 federnd verformbar.

Ferner hat jede Weichkomponentenanordnung 28 eine Innen-Weichkomponente 32, die sich an die zugehörige Übergangs-Weichkomponente 30 anschließt und innerhalb des Umrandungsstegs 27 angeordnet ist. Die Innen-Weichkomponente 32 verläuft von dem gelenkseitigen Ende 13 bis zu dem freien Ende 14. Sie erstreckt sich auch in die Durchgangsaussparung 25 hinein. Der Brillenbügel 2 ist so über seine gesamte Länge federnd verformbar.

Ferner hat jede Weichkomponentenanordnung 28 eine Außen-Weichkomponente 33, die innerhalb der Verbindungsnut 26 angeordnet ist. Die Außen-Weichkomponente 33 erstreckt sich auch in die Durchgangsaussparung 25 hinein. Sie ermöglicht eine federnde Verformung des Brillenbügels 2 über seine gesamte Länge.

Die Außen-Weichkomponente 33 steht mit der Innen-Weichkomponente 32 über die jeweilige Verbindungsöffnung 23 in Verbindung, die von einer Zwischen-Weichkomponente 34 der jeweiligen Weichkomponentenanordnung 28 vollständig ausgefüllt ist.

Die Zwischen-Weichkomponente 34 greift dabei randseitig bei dem Endbereich 31 in den jeweiligen Brillenbügel-Gelenkkörper 17 und in das Brillenbügel-Hauptteil 38 ein. Die Zwischen-Weichkomponenten-Aussparung 22 ist so ausgefüllt. Der Brillenbügel 2 ist so bei dem Brillenbügel-Gelenkkörper 17 federnd verformbar.

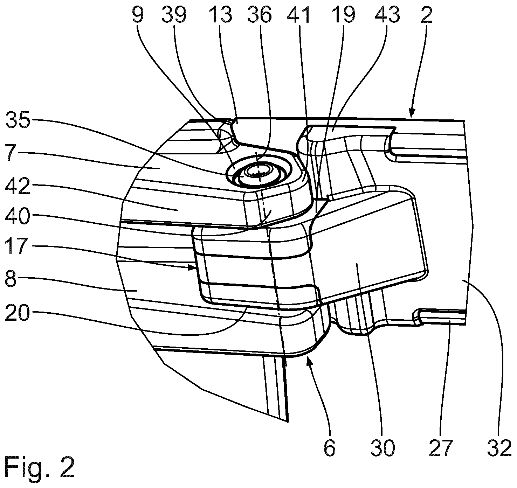

Wie beispielsweise Fig. 2 zeigt, hat die Brillen-Scheibenanordnung 1 bei jeder Brillenbügel-Lageranordnung 6 außenseitig einen Schwenkanschlag 39. Vorzugsweise ist jeder Schwenkanschlag 39 an dem jeweiligen ersten Scharnieransatz 7 seitlich ausgebildet.

Ferner weist die Brillen-Scheibenanordnung 1 bei jeder Brillenbügel-Lageranordnung 6 eine freie Bügelschlagkurve 40 auf, die bevorzugt zumindest an dem jeweiligen ersten Scharnieransatz 7 ausgebildet ist und benachbart zu dem Schwenkanschlag 39 endet/beginnt. Jede Bügelschlagkurve 40 ist entgegen einer Blickrichtung der Brille gewandt. Sie läuft bereichsweise beabstandet um die erste Lageröffnung 9 und ist konvex gekrümmt. Jede Bügelschlagkurve 40 weicht in ihrem Verlauf von einem Kreisbogen ab und hat einen im Wesentlichen zentralen Scheitel 41, der beabstandet zu einem der Symmetrieebene 3 zugewandten inneren Endbereich 42 des jeweiligen ersten Scharnieransatzes 7 und dem Schwenkanschlag 39 ausgebildet ist.

Jeder Brillenbügel 2 hat an seinem gelenkseitigen Endbereich einen Anlagevorsprung 43, der Bestandteil der jeweiligen Weichkomponentenanordnung 28 ist.

In montiertem Zustand der Brille greift ein Gelenkstift 35 unter Bildung einer Schwenkachse 36 in die Lageröffnungen 9, 10 und die zugehörige Gelenkmittelaufnahme 21 unter Bildung eines Brillenbügel-Gelenks bzw. - Scharniers ein. Der Gelenkstift 35 ist axial festgelegt.

In montiertem Zustand der Brille greift jeder Brillenbügel-Gelenkkörper 17 in die jeweilige Brillenbügel-Gelenkkörper-Aussparung ein, sodass der Brillenbügel 2 um die Schwenkachse 36 zwischen einer vollständig eingeklappten Stellung und einer vollständig ausgeklappten Stellung verschwenkbar ist. Die Kopffläche 19 ist benachbart zu dem ersten Scharnieransatz 7 angeordnet, während die Fußfläche 20 benachbart zu dem zweiten Scharnieransatz 8 angeordnet ist.

Jeder Brillenbügel 2 ist biegsam bzw. federnd ausgeführt. Insbesondere ist jeder Brillenbügel 2 direkt in dem Brillenbügel-Gelenkkörper 17 und unmittelbar benachbart zu diesem biegsam bzw. federnd ausgestaltet. Dies wird insbesondere durch die Gelenkkörper-Weichkomponente 29 und die Übergangs-Weichkomponente 30 sowie die Zwischen-Weichkomponente 34 erreicht.

Die Innen-Weichkomponente 32 und die Außen-Weichkomponente 33 sorgen für eine federnde Ausbildung des Brillenbügels 2 über dessen, insbesondere gesamte, Längserstreckung.

Wenn die Brille von einem Träger mit einem besonders breiten Kopf getragen wird, kommt es insbesondere zu einer Aufspreizung zwischen dem Brillenbügel-Gelenkkörper 17 und dem zugehörigen Brillenbügel-Hauptteil 38. Dabei erfährt die Weichkomponentenanordnung 28 dort, insbesondere die Übergangs-Weichkomponente 30 und die Zwischen-Weichkomponente 34, eine elastische Dehnung. Dabei wirkt die Weichkomponentenanordnung 28 insbesondere dort dieser Aufspreizung entgegen und wirkt für einen rückfedernden Effekt des Brillenbügels 2 in Richtung auf eine nicht-aufgebogene bzw. ursprüngliche Brillenbügel-Tragestellung. Ferner wird der Brillenbügel-Gelenkkörper 17 elastisch aufgebogen. Der Brillenbügel 2 wird so aus seiner nicht-aufgebogenen bzw. ursprünglichen Brillenbügel-Tragestellung federnd nach außen ausgelenkt, insbesondere unter Überwindung einer Federkraft. Dabei wird eine Rückführkraft durch die Weichkomponentenanordnung erzeugt. Der Brillenbügel 2 kann sich ferner über seine gesamte Länge elastisch aufbiegen. Der Brillenbügel 2 wird homogen seitlich gegen den Kopf des Trägers gedrückt.

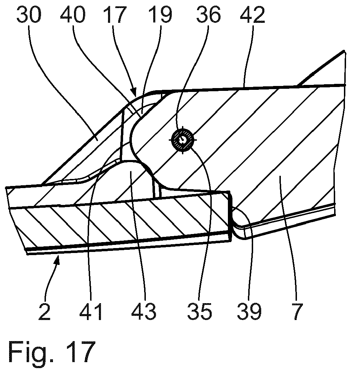

Wenn sich ein Brillenbügel 2 in seiner vollständig ausgeklappten Tragestellung entsprechend Fig. 17 befindet, liegt der jeweilige Brillenbügel-Grundkörper 11 an dem Schwenkanschlag 39 an. Der Schwenkanschlag 39 verhindert ein weiteres Verschwenken des jeweiligen Brillenbügels 2 nach seitlich außen.

Ferner liegt dann der Anlagevorsprung 43, insbesondere mit einer Seitenflanke und/oder einem Kopfbereich, zwischen dem Schwenkanschlag 39 und dem Scheitel 41 an der Brillenbügelkurve 40 fest drückend an. Einem Verschwenken des Brillenbügels 2 in seine eingeklappte Transportstellung ist so ein Widerstand entgegengesetzt.

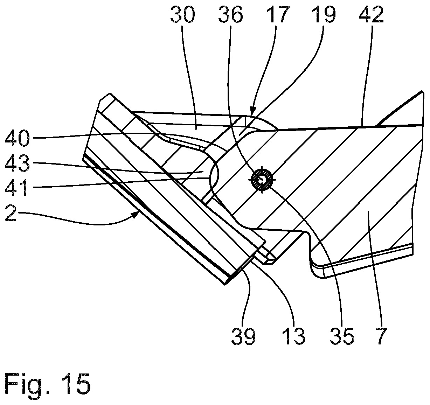

Bei manueller Überwindung dieses Widerstands gleitet der Anlagevorsprung 43 durch Verschwenken des Brillenbügels 2 an der Bügelschlagkurve 40 in Richtung auf den Scheitel 41 entlang. Der Anlagevorsprung 43 erreicht dann durch Verschwenken des Brillenbügels 2 die in Fig. 15 gezeigte Zwischenstellung. Bei dem Scheitel 41 liegt quasi eine maximale Geometrieüberlagerung zwischen der Bügelschlagkurve 40 und dem Anlagevorsprung 43 vor, die in Fig. 15 veranschaulicht ist. Dabei wird der Anlagevorsprung 43 insbesondere geringfügig verformt.

Vor Erreichen des Scheitels 41 kehrt bei nicht ausreichender manueller Schwenkkraft der Brillenbügel 2 automatisch stets in seine vollständig ausgeklappte Tragestellung zurück, was auf den vorherrschenden Druck zwischen der Bügelschlagkurve 40 und dem Anlagevorsprung 43 zurückzuführen ist. Der Anlagevorsprung 43 gleitet dabei an der Bügelschlagkurve 40 entlang bzw. zurück, was zu der entsprechenden Schwenkbewegung des Brillenbügels 2 führt.

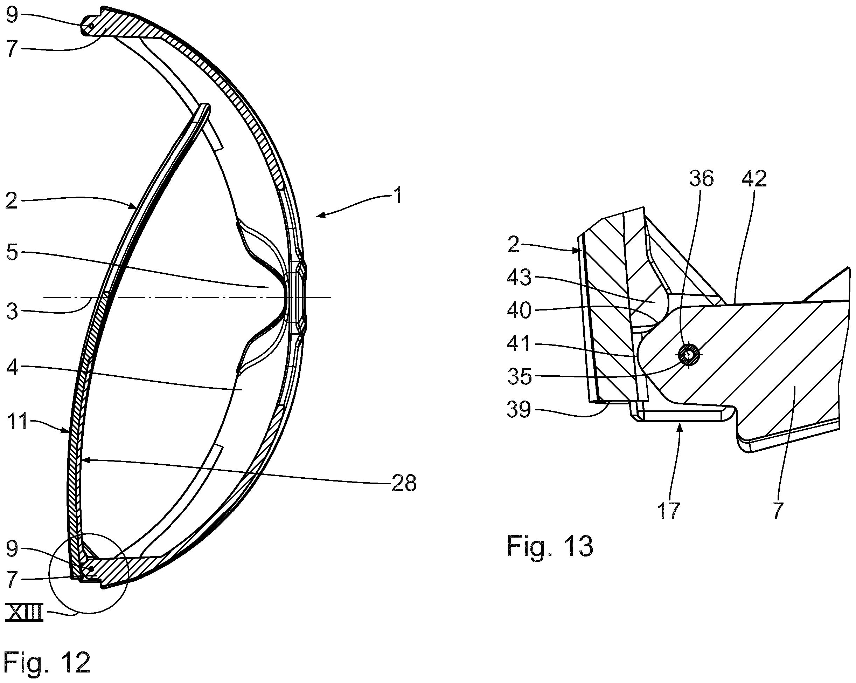

Nachdem der Anlagevorsprung 43 den Scheitel 41 passiert hat, bewegt sich der Brillenbügel 2 automatisch in seine vollständig eingeklappte Transportstellung (Fig. 13), was wiederum auf den vorherrschenden Druck zwischen der Bügelschlagkurve 40 und dem Anlagevorsprung 43 zurückzuführen ist. Der Anlagevorsprung 43 gleitet dabei an der Bügelschlagkurve 40 entlang, was zu der entsprechenden Schwenkbewegung des Brillenbügels 2 führt. Bei dem Scheitel 41 liegt so ein Totpunkt des Bügelschlags vor. Der Brillenbügel 2 wird in keine Endstellung gezwängt.

In der vollständig eingeklappten Transportstellung befindet sich der Anlagevorsprung 43 benachbart zu dem inneren Endbereich 42 an der Bügelschlagkurve 40. Analoges gilt für ein umgekehrtes Verschwenken des Brillenbügels 2. Die Brille hat demnach einen Bügelschlag.

Zur Herstellung des Brillenbügels 2 wird die Weichkomponentenanordnung 28 in mindestens einem Spritzgießschritt an den Brillenbügel-Grundkörper 11 unlösbar angespritzt. Jeder Brillenbügel 2 ist ein Zweikomponenten-Brillenbügel.

## Patentansprüche

**1.** Brillenbügel für eine Brille,

a) mit einem gelenkseitigen Ende (13) und einem dem gelenkseitigen Ende (13) gegenüberliegenden freien Ende (14),
b) mit einer Innenseite (15) zur benachbarten Anordnung zu einem Kopf eines Trägers der Brille beim Tragen derselben und einer der Innenseite (15) gegenüberliegenden Außenseite (16),
c) mit einem aus einem ersten Material gebildeten Brillenbügel-Grundkörper (11), der aufweist

i) ein sich längs einer Längsrichtung (12) des Brillenbügels (2) erstreckendes Brillenbügel-Hauptteil (38), das zwischen dem gelenkseitigen Ende (13) und dem freien Ende (14) verläuft, und
ii) einen benachbart zu dem gelenkseitigen Ende (13) des Brillenbügels (2) an dem Brillenbügel-Hauptteil (38) angeordneten Brillenbügel-Gelenkkörper (17) mit einer Gelenkmittelaufnahme (21) zur gelenkigen Verbindung mit einer Brillen-Scheibenanordnung (4), und

d) mit einer Weichkomponentenanordnung (28), die

i) aus einem zweiten Material gebildet ist, das weicher als das erste Material ist,
ii) eine an der Innenseite (15) des Brillenbügels (2) an dem Brillenbügel-Gelenkkörper (17) angeordnete federnde Gelenkkörper-Weichkomponente (29) zum Ermöglichen eines federnden Aufbiegens des Brillenbügels (2) bei dem Brillenbügel-Gelenkkörper (17) umfasst, und
iii) zusammenhängend ist,

**dadurch gekennzeichnet, dass**
e) die Gelenkkörper-Weichkomponente (29) in mindestens einer Brillenbügel-Gelenkkörper-Nut (18) des Brillenbügel-Gelenkkörpers (17) angeordnet ist, und
f) die Weichkomponentenanordnung (28) aufweist

i) eine Zwischen-Weichkomponente (34), die bereichsweise zwischen dem Brillenbügel-Gelenkkörper (17) und dem Brillenbügel-Hauptteil (38) angeordnet ist, und
ii) eine Außen-Weichkomponente (33), die an dem Brillenbügel-Hauptteil (38) angeordnet ist und sich von dem gelenkseitigen Ende (13) des Brillenbügels (2) in Richtung auf das freie Ende (14) des Brillenbügels (2) an der Außenseite (16) des Brillenbügels (2) erstreckt,
iii) wobei die Zwischen-Weichkomponente (34) und die Außen-Weichkomponente (33) benachbart zu dem Brillenbügel-Gelenkkörper (17) zum Ermöglichen eines Aufbiegens des Brillenbügels (2) zusammenhängend sind.

**2.** Brillenbügel nach Anspruch 1, **dadurch gekenn-**

**zeichnet, dass** die Gelenkkörper-Weichkomponente (29) beabstandet zu einer Kopffläche (19) und/oder Fußfläche (20) des Brillenbügel-Gelenkkörpers (17) angeordnet ist.

**3.** Brillenbügel nach Anspruch 1 oder 2, **dadurch gekennzeichnet, dass** sich die Gelenkkörper-Weichkomponente (29) in der Längsrichtung (12) des Brillenbügels (2) über den gesamten Brillenbügel-Gelenkkörper (17) erstreckt.

**4.** Brillenbügel nach einem der vorherigen Ansprüche, **dadurch gekennzeichnet, dass** die Weichkomponentenanordnung (28) eine, insbesondere federnde, Übergangs-Weichkomponente (30) aufweist, die sich an einen dem gelenkseitigen Ende (13) des Brillenbügels (2) abgewandten Endbereich (31) des Brillenbügel-Gelenkkörpers (17) anschließt und von dort in Richtung auf das Brillenbügel-Hauptteil (38) zum Ermöglichen eines, insbesondere federnden, Aufbiegens des Brillenbügels (2) verläuft.

**5.** Brillenbügel nach einem der vorherigen Ansprüche, **dadurch gekennzeichnet, dass** die Zwischen-Weichkomponente (34) federnd ist.

**6.** Brillenbügel nach einem der vorherigen Ansprüche, **dadurch gekennzeichnet, dass** die Zwischen-Weichkomponente (34) bereichsweise zwischen dem Brillenbügel-Gelenkkörper (17) und dem Brillenbügel-Hauptteil (38) an einem dem gelenkseitigen Ende (13) des Brillenbügels (2) abgewandten Endbereich (31) des Brillenbügel-Gelenkkörpers (17) zum Ermöglichen eines, insbesondere federnden, Aufbiegens des Brillenbügels (2) angeordnet ist.

**7.** Brillenbügel nach einem der vorherigen Ansprüche, **dadurch gekennzeichnet, dass** die Zwischen-Weichkomponente (34) und die Außen-Weichkomponente (33) benachbart zu dem Brillenbügel-Gelenkkörper (17) zum Ermöglichen eines federnden Aufbiegens des Brillenbügels (2) zusammenhängend sind.

**8.** Brillenbügel nach einem der vorherigen Ansprüche, **gekennzeichnet durch** mindestens eine in dem Brillenbügel-Hauptteil (38) ausgebildete Verbindungsöffnung (23), über welche die Zwischen-Weichkomponente (34) und die Außen-Weichkomponente (33) miteinander in Verbindung stehen.

**9.** Brillenbügel nach Anspruch 8, **dadurch gekennzeichnet, dass** die mindestens eine Verbindungsöffnung (23) benachbart zu dem Brillenbügel-Gelenkkörper (17) in dem Brillenbügel-Hauptteil (38) ausgebildet ist.

**10.** Brillenbügel nach einem der vorherigen Ansprüche, **dadurch gekennzeichnet, dass** die Weichkomponentenanordnung (28) eine Innen-Weichkomponente (32) aufweist, die an dem Brillenbügel-Hauptteil (38) angeordnet ist und sich von dem Brillenbügel-Gelenkkörper (17) in Richtung auf das freie Ende (14) des Brillenbügels (2) an der Innenseite (15) des Brillenbügels (2) erstreckt.

**11.** Brillenbügel nach einem der vorherigen Ansprüche, **dadurch gekennzeichnet, dass** das erste Material eine erste Härte H1 und das zweite Material eine zweite Härte H2 aufweist, wobei gilt: $1{,}5 \leq H1/H2 \leq 5$.

**12.** Brille, umfassend zwei Brillenbügel (2) nach einem der vorherigen Ansprüche.

## Claims

**1.** Temple for spectacles,

a) having a hinge-side end (13) and a free end (14) opposite to the hinge-side end (13),
b) having an inner side (15) for arranging next to a head of a wearer of the spectacles when wearing the latter, and an outer side (16) opposite to the inner side (15),
c) having a temple basic body (11) formed from a first material, said basic body having

i) a temple main part (38) that extends in a longitudinal direction (12) of the temple (2) and extends between the hinge-side end (13) and the free end (14), and
ii) a temple hinge body (17), arranged on the temple main part (38) next to the hinge-side end (13) of the temple (2), having a hinge-means receptacle (21) for connecting in an articulated manner to a spectacle lens arrangement (4), and

d) having a flexible-component arrangement (28) which

i) is formed from a second material that is more flexible than the first material,
ii) comprises a resilient hinge-body flexible component (29), arranged on the inner side (15) of the temple (2) on the temple hinge body (17), for allowing resilient bending out of the temple (2) at the temple hinge body (17), and
iii) is cohesive,

**characterized in that**
e) the hinge-body flexible component (29) is arranged in at least one temple hinge-body groove (18) in the temple hinge body (17), and

f) the flexible-component arrangement (28) has

i) an intermediate flexible component (34), which is arranged regionally between the temple hinge body (17) and the temple main part (38), and

ii) an outer flexible component (33), which is arranged on the temple main part (38) and extends from the hinge-side end (13) of the temple (2) in the direction of the free end (14) of the temple (2) on the outer side (16) of the temple (2),

iii) wherein the intermediate flexible component (34) and the outer flexible component (33) are cohesive next to the temple hinge body (17) to allow the temple (2) to be bent out.

**2.** Temple according to Claim 1, **characterized in that** the hinge-body flexible component (29) is arranged at a distance from a top face (19) and/or bottom face (20) of the temple hinge body (17).

**3.** Temple according to Claim 1 or 2, **characterized in that** the hinge-body flexible component (29) extends in the longitudinal direction (12) of the temple (2) along the entire temple hinge body (17).

**4.** Temple according to one of the preceding claims, **characterized in that** the flexible-component arrangement (28) has an, in particular resilient, transitional flexible component (30), which adjoins an end region (31) of the temple hinge body (17) that is remote from the hinge-side end (13) of the temple (2), and extends from there in the direction of the temple main part (38) in order to allow the temple (2) to be bent out, in particular in a resilient manner.

**5.** Temple according to one of the preceding claims, **characterized in that** the intermediate flexible component (34) is resilient.

**6.** Temple according to one of the preceding claims, **characterized in that** the intermediate flexible component (34) is arranged regionally between the temple hinge body (17) and the temple main part (38) in an end region (31), remote from the hinge-side end (13) of the temple (2), of the temple hinge body (17) in order to allow the temple (2) to be bent out, in particular in a resilient manner.

**7.** Temple according to one of the preceding claims, **characterized in that** the intermediate flexible component (34) and the outer flexible component (33) are cohesive next to the temple hinge body (17) in order to allow the temple (2) to be bent out in a resilient manner.

**8.** Temple according to one of the preceding claims, **characterized by** at least one connecting opening (23) which is formed in the temple main part (38) and via which the intermediate flexible component (34) and the outer flexible component (33) are connected with each other.

**9.** Temple according to Claim 8, **characterized in that** the at least one connecting opening (23) is formed in the temple main part (38) next to the temple hinge body (17).

**10.** Temple according to one of the preceding claims, **characterized in that** the flexible-component arrangement (28) has an inner flexible component (32) which is arranged on the temple main part (38) and extends on the inner side (15) of the temple (2) from the temple hinge body (17) in the direction of the free end (14) of the temple (2).

**11.** Temple according to one of the preceding claims, **characterized in that** the first material has a first hardness H1 and the second material has a second hardness H2, wherein: $1.5 \leq H1/H2 \leq 5$.

**12.** Spectacles comprising two temples (2) according to one of the preceding claims.

## Revendications

**1.** Branche de lunettes pour une paire de lunettes,

a) pourvue d'une extrémité (13) côté articulation et d'une extrémité (14) libre opposée à l'extrémité (13) côté articulation,

b) pourvue d'une face intérieure (15) destinée à être disposée de manière adjacente à la tête d'un porteur de lunettes lorsqu'il porte celles-ci, et d'une face extérieure (16) opposée à la face intérieure (15),

c) pourvue d'un corps de base de branche de lunettes (11) constitué d'un premier matériau qui comporte

i) une partie principale (38) de branche de lunettes s'étendant le long d'une direction longitudinale (12) de la branche de lunettes (2) et s'étendant entre l'extrémité (13) côté articulation et l'extrémité (14) libre, et comprend

ii) un corps d'articulation (17) de branche de lunettes disposé sur la partie principale (38) de branche de lunettes, de manière adjacente à l'extrémité d'articulation (13) de branche de lunettes (2), et pourvu d'un logement pour articulation (21) pour la liaison articulée à une façade (4) de lunettes, et

d) pourvue d'un système à composant souple (28)

i) constitué d'un second matériau qui est plus souple que le premier matériau,
ii) un composant souple (29) pour corps d'articulation, ledit composant souple étant élastique et disposé sur la face intérieure (15) de la branche de lunettes (2) au niveau du corps d'articulation (17) de branche de lunettes afin de permettre la flexion élastique de la branche de lunettes (2) au niveau du corps d'articulation (17) de branche de lunettes, et
iii) est continue, **caractérisée en ce que**

e) le composant souple (29) pour corps d'articulation est disposé dans au moins une rainure (18) de corps d'articulation (17) de branche de lunettes, et **en ce que**
f) le système à composant souple (28) comporte

i) un composant souple intermédiaire (34) disposé, par endroits, entre le corps d'articulation (17) de branche de lunettes et la partie principale (38) de branche de lunettes, et
ii) un composant souple extérieur (33) qui est disposé au niveau de la partie principale (38) de branche de lunettes et qui s'étend de l'extrémité (13) côté articulation de la branche de lunettes (2) en direction de l'extrémité (14) libre de la branche de lunettes (2) sur la face extérieure (16) de la branche de lunettes (2),
iii) le composant souple intermédiaire (34) et le composant souple extérieur (33) adjacent au corps d'articulation (17) de branche de lunettes étant continus afin de permettre la flexion de la branche de lunettes (2).

2. Branche de lunettes selon la revendication 1, **caractérisée en ce que** le composant souple (29) pour corps d'articulation est disposé à distance d'une surface de tête (19) et/ou d'une surface de pied (20) du corps d'articulation (17) de branche de lunettes.

3. Branche de lunettes selon la revendication 1 ou 2, **caractérisée en ce que** le composant souple (29) pour corps d'articulation s'étend dans la direction longitudinale (12) de la branche de lunettes (2) sur l'ensemble du corps d'articulation (17) de branche de lunettes.

4. Branche de lunettes selon l'une des revendications précédentes, **caractérisée en ce que** le système à composant souple (28) comporte un composant souple de transition (30), en particulier élastique, qui se lie à une zone d'extrémité (31) du corps d'articulation (17) de branche de lunettes opposée à l'extrémité (13) côté articulation de la branche de lunettes (2) et s'étend à partir de là en direction de la partie principale (38) de branche de lunettes afin de permettre la flexion, en particulier élastique, de la branche de lunettes (2).

5. Branche de lunettes selon l'une des revendications précédentes, **caractérisée en ce que** le composant souple intermédiaire (34) est élastique.

6. Branche de lunettes selon l'une des revendications précédentes, **caractérisée en ce que** le composant souple intermédiaire (34) est disposé, par endroits, entre le corps d'articulation (17) de branche de lunettes et la partie principale (38) de branche de lunettes dans une zone d'extrémité (31) du corps d'articulation (17) de branche de lunettes qui est opposée à l'extrémité d'articulation (13) de branche de lunettes (2) afin de permettre la flexion, en particulier élastique, de la branche de lunettes (2).

7. Branche de lunettes selon l'une des revendications précédentes, **caractérisée en ce que** le composant souple intermédiaire (34) et le composant souple extérieur (33) adjacent au corps d'articulation (17) de branche de lunettes sont continus afin de permettre la flexion élastique de la branche de lunettes (2).

8. Branche de lunettes selon l'une des revendications précédentes, **caractérisée par** au moins une ouverture de liaison (23) réalisée dans la partie principale (38) de branche de lunettes, ouverture par l'intermédiaire de laquelle le composant souple intermédiaire (34) et le composant souple extérieur (33) sont reliés.

9. Branche de lunettes selon la revendication 8, **caractérisée en ce qu'**au moins une ouverture de liaison (23) est réalisée dans la partie principale (38) de branche de lunettes, de manière adjacente au corps d'articulation (17) de branche de lunettes.

10. Branche de lunettes selon l'une des revendications précédentes, **caractérisée en ce que** le système à composant souple (28) comporte un composant souple intérieur (32) qui est disposé au niveau de la partie principale (38) de branche de lunettes et qui s'étend à partir du corps d'articulation (17) de branche de lunettes en direction de l'extrémité (14) libre de la branche de lunettes (2) sur la face intérieure (15) de la branche de lunettes (2).

11. Branche de lunettes selon l'une des revendications précédentes, **caractérisée en ce que** le premier matériau présente une première dureté H1 et **en ce que** le second matériau présente une seconde dureté H2, dans laquelle ce qui suit s'applique :

$$1,5 \leq H1/H2 \leq 5.$$

**12.** Lunettes comprenant deux branches de lunettes (2) selon l'une des revendications précédentes.

14
12
2
11
15
28
II
13
6
7
8
9
6
10
1
4
5
7
8

Fig. 1

2
43
19
41
36
13
39
9
32
27
30
6
35
7
42
40
17
8
20

Fig. 2

1
9
7
4
5
3
7
9
6
15
2
14
16
Fig. 3

14
16
25
11
38
26
23
13
17

Fig. 4

15
38
24
22
31
21
17
14
16
19
13

Fig. 5

14
25
15
38
27
22
31
18
20
19
13
17

Fig. 6

14
16
2
25
11
38
26
33
13
17

Fig. 7

2
32
38
15
24
27
30
31
28
21
17
14
16
33
43
13
19

Fig. 8

15
2
25
38
27
32
30
31
18
17
14
13
19

Fig. 9

1
10
8
4
5
3
8
6
29
10
30
28
32
2
15
14
16
34
23
33
XI

Fig. 10

8
4
29
21
34
17
23
30
33
11
2
32

Fig. 11

2
43
40
41
35
39
36
42
7
17

Fig. 13

1
28
9
7
2
5
3
4
11
9
7
XIII

Fig. 12

1
9
7
4
5
3
15
7
9
14
2
16
XV
13
Fig. 14

42
19
17
36
30
7
35
13
39
40
43
41
2

Fig. 15

1
7
6
9
XVII
33
28
32
2
15
14
16
9
7
4
5
3

Fig. 16

42
36
19
17
40
30
7
39
35
43
41
2

Fig. 17

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102017206431
- US 7165838 B1
- US 2016033790 A1
- EP 0457026 A2
- US 20090059160 A1
- US 2761353 A
- US 2684014 A
- DE 102005014512 A1